# EUROPÄISCHE PATENTSCHRIFT

(11) **EP 1 741 695 B1**
(45) Veröffentlichungstag und Bekanntmachung des Hinweises auf die Patenterteilung: **15.07.2009**
(21) Anmeldenummer: 06009179.0
(22) Anmeldetag: 04.05.2006
(51) Int. Cl.: C07C 45/52

(54) **Verfahren zur Herstellung von hochreinem, halogenfreien o-Phthalaldehyd**
Process for producing high purity, halogen-free o-phthalaldehyde
Procédé de fabrication de o-phthalaldehyde de haute pureté exempte d'halogène

(30) Priorität: 28.06.2005 AT 10872005
(43) Veröffentlichungstag der Anmeldung: 10.01.2007
(73) Patentinhaber: DSM Fine Chemicals Austria Nfg GmbH & Co KG, 4021 Linz (AT)
(72) Erfinder: Giselbrecht, Karlheinz, 4061 Pasching (AT); Reiter, Klaus, 4020 Linz (AT); Hermanseder, Rudolf, 4624 Pennewang (AT)
(74) Vertreter: Habets, Winand

(56) Entgegenhaltungen:
- EP-A1- 0 522 312
- DE-A1- 3 108 790
- US-A- 5 107 032

## Beschreibung

Phthalaldehyde, wie o-Phthaldialdehyd (OPA), finden in vielen Gebieten beispielsweise als Zwischenprodukte für die Herstellung von Farbstoffen, optischen Aufhellern oder speziellen Polymeren, in der Biozid- oder Fotoindustrie, sowie für die Synthese von Pharmachemikalien Anwendung. Aus diesem Grund sind bereits mehrere Herstellungsvarianten beschrieben. So kann der o-Phthaldialdehyd (OPA) beispielsweise gemäß EP-B-0 147 593 durch Ozonolyse von Naphthalin in Methanol und katalytischer Reduktion der dabei entstehenden Peroxide mit anschließender Extraktion bzw. Kristallisation gewonnen werden. Der Nachteil bei diesem Verfahren ist, dass der sich als Nebenprodukt bildende Ester nur schwer und unzureichend vom OPA abgetrennt werden kann.

Weiters stellt OPA eine reaktive Verbindung dar, die thermisch und oxidativ nicht stabil ist und die bei längerer Lagerung zum Verblocken, wodurch langwierige Löseprozesse erforderlich werden, die eventuell zu einer Verfärbung von OPA führen können.

Um den Aldehyd vor ungewollten Umsetzungen zu schützen, wurde in EP-A1-0522 312 die Möglichkeit beschrieben, o-Phthaldialdehydtetraalkylacetale, hergestellt durch elektrochemische Oxidation, als Depotverbindungen einzusetzen.

Aus EP-B-0 839 789 ist weiters bekannt OPA durch säurekatalysierte Acetalbildung mit anschließender Destillation in eine geeignete Depotverbindung, wie etwa in ein Dialkoxyphthalan oder Tetraalkylacetal, zu überführen, woraus im Bedarfsfall nach vollständiger Acetalspaltung durch saure Hydrolyse rohes OPA mit Reinheiten von über 99,5% erhalten wird.

Da rohes OPA, beispielsweise hergestellt nach EP-B-0 839 789, jedoch eine rot orange Farbe aufinreist, muss anschließend, nach Entfärben mit beispielsweise Aktivkohle oder Tonsil, noch umkristallisiert werden.

Das feine OPA-Pulver mit einem Schmelzpunkt von 57°C, das durch Kristallisation erhalten wird, tendiert ebenfalls zum Verblocken. Weiters weisen die verschiedenen Chargen keine gleichbleibende Farbe und Qualität auf.

Aus US 5,107,032 ist die Herstellung von OPA durch Herstellung eines Tetrahalo-o-xylols mit anschließender Hydrolyse bei 90 bis 146°C bekannt. Dabei wird beispielsweise Tetrachloro-o-xylol mit Natriumacetat in wässriger Essigsäure bei maximal 146°C und 3,5 bar verseift, worauf nach 5-maliger Extraktion mit Toluol, anschließender Destillation der vereinten organischen Phasen OPA in 87%-iger Ausbeute isoliert wird. Nachteilig bei diesem Verfahren ist jedoch unter anderem, dass das erhaltene Produkt nicht halogenfrei ist.

Aufgabe der vorliegenden Erfindung war es daher ein verbessertes Verfahren zur Herstellung von hochreinem, halogenfreien OPA zu finden.

Unerwarteterweise konnte diese Aufgabe durch Hydrolyse von Tetrahalo-o-xylol bei hoher Temperatur über 155°C, anschließender Acetalisierung zum korrespondierenden o-Phthalaldehydacetal, destillativer Reinigung des Acetals, sowie Acetalspaltung bei pH >1,5 gelöst werden.

Gegenstand der Erfindung ist demnach ein verbessertes Verfahren zur Herstellung von hochreinem, halogenfreien o-Phthalaldehyd, das dadurch gekennzeichnet ist, dass
a) Tetrahalo-o-xylol bei einer Temperatur von 155 - 160°C und einem Druck von 2 bis 5 bar, gegebenenfalls in Anwesenheit eines Phasentransferkatalysators, zu o-Phthalaldehyd hydrolysiert wird, der
b) in einer sauren, alkoholischen Lösung bei einer Temperatur von 0 bis Rückflusstemperatur in das korrespondierende Dialkoxyphthalan überführt und anschließend
c) eine Acetalspaltung durch saure Hydrolyse bei einem pH >1,5 bis pH 7 erfolgt, wodurch hochreiner, halogenfreier o-Phthalaldehyd erhalten wird.

Bei dem erfindungsgemäßen Verfahren wird hochreiner, halogenfreier o-Phthalaldehyd, (OPA) hergestellt.

Als Ausgangsmaterial dient ein Tetrahalo-o-xylol. Geeignete Tetrahalo-o-xylole sind beispielsweise solche der Formel 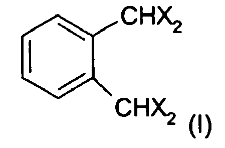 in der X Cl, Br oder I sein kann.
Das Tetrahalo-o-xylol (THX) kann dabei gegebenenfalls ein- oder mehrfach durch geeignete Reste substituiert sein. Geeignete Reste sind beispielsweise C₁-C₄-Alkyl, C₅-C₂₀-Aryl, OH, NO₂, CN, Cl, Br oder CO₂H.
Bevorzugt werden unsubstituierte THX-Verbindungen eingesetzt, X bedeutet bevorzugt Chlor.
Geeignete THX-Verbindungen sind kommerziell erhältlich (mit X gleich Cl oder Br) oder auf bekannte Weise, beispielsweise durch Reaktion von o-Xylol mit elementarem Chlor unter Lichteinwirkung oder mit Hilfe von Radikalstartern wie AIBN, PCl3, und Dibenzoylperoxid u.s.w. herstellbar.

Erfindungsgemäß wird ein THX bei einer Temperatur von 155 - 160°C und einem Druck von 2 bis 5 bar, bevorzugt bei 3 bis 4 bar, zum entsprechenden o-Phthalaldehyd hydrolysiert.
Die Hydrolyse wird dabei in einem wässerigen System aus C₁-C₄-Carbonsäure in Gegenwart einer Base, wie etwa NaOH, LiOH, KOH, u.s.w., durchgeführt. Bevorzugt wird Essigsäure in Gegenwart von NaOH (40 bis 50%ig) eingesetzt.
Dazu wird bevorzugt die Carbonsäure bzw. wässerige Carbonsäure und ein THX vorgelegt und anschließend die wässerige Base und Wasser zugesetzt. Die Reihenfolge der Zugabe kann jedoch auch variieren.
Die Menge an eingesetzter Carbonsäure liegt bei > 4 bis 20 Moläquivalenten, bezogen auf THX. Bevorzugt bei 8 bis 10 Moläquivalenten, bezogen auf THX..
Die Menge an eingesetzter Base liegt bei 4,0 bis 5,0 Moläquivalenten, bezogen auf THX.

Gegebenenfalls kann dem Reaktionsgemisch, um eine höhere Reaktionsgeschwindigkeit zu erzielen, ein Phasentransferkatalysator (PTK) zugesetzt werden (1-5 mol% bezogen auf TCX). Geeignete PTKs sind übliche Verbindungen, wie etwa quaternäre Ammoniumsalze und Phosphoniumsalze, beispielsweise Tetraalkylammonium- und

Tetraalkylphosphoniumsalze oder Arylalkyl-trialkylammoniumsalze. Bevorzugte Salze sind dabei Halogenide. Beispiele dafür sind Tetrabutylammoniumchlorid oder - bromid, Ethyl-trioctylphosphoniumchlorid, Benzyltriethylammoniumchlorid, u.s.w.

Nach erfolgter Hydrolyse wird der entsprechende rohe o-Phthalaldehyd (OPA) durch Extraktion mit üblichen Extraktionsmitteln, wie etwa Methyl-tert.-butylether, Toluol, Essigester, u.s.w., und anschließender Destillation der Lösungs- und Extraktionsmittel erhalten.

Anschließend erfolgt in Schritt b) die Überführung des rohen OPAs in das Acetal, das Dialkoxyphthalan, auf übliche Weise durch Säure katalysierte Acetalisierung mit einem Alkohol.
Als Alkohol wird dabei bevorzugt ein C₁-C₄-Alkohol, besonders bevorzugt Methanol oder Ethanol eingesetzt.
Der rohe OPA wird dazu in dem Alkohol gelöst. Anschließend wird die Lösung durch Zugabe einer Säure auf einen pH-Wert zwischen 0 und 3, bevorzugt zwischen 0,5 und 2, eingestellt.
Als Säuren eignen sich beispielsweise Mineralsäuren, wie etwa HCl, H₂SO₄, H₃PO₄, organische Säuren, wie etwa Ameisensäure, Essigsäure, p-Toluolsulfon- oder Methansulfonsäure oder saure lonentauscher.
Die Temperatur bei diesem Schritt beträgt 0°C bis Rückflusstemperatur, bevorzugt bis 50°C.

Nach erfolgter Acetalisierung wird die Lösung zur Neutralisierung der Säure bzw. saurer organischen Verbindungen mit einer wässrigen Lauge versetzt. Als Lauge eignen sich beispielsweise NaOH oder KOH.

Anschließend oder gleichzeitig wird der als Lösungsmittel verwendete Alkohol abdestilliert.
Die Isolierung des Dialkoxyphthalans erfolgt wiederum durch Extraktion und nachfolgende Destillation.
Die isolierten OPA-Acetale werden dabei in sehr hoher, halogenfreier Qualität (>99,5 GC FI%) und in sehr hoher Ausbeute von bis zu über 92% erhalten.

Die Spaltung der Acetale erfolgt in Schritt c) ebenfalls auf üblichen Weg, analog dem Stand der Technik, über saure Hydrolyse.
Der pH-Wert liegt erfindungsgemäß >1,5 bis 7, bevorzugt bei 1,6 bis 2,5.
Als Säuren werden wiederum Mineralsäuren wie HCl, H₂SO₄, H₃PO₄ oder organischer Säuren wie Essigsäure, Ameisensäure und p-Toluolsulfon- oder Methansulfonsäure eingesetzt.
Die Reaktionstemperatur liegt bevorzugt zwischen Raumtemperatur und 60°C. Gleichzeitig wird der sich abspaltende Alkohol und gegebenenfalls die Säure abdestilliert.
Der so erhaltene OPA kann sodann gegebenenfalls noch weiter, beispielsweise durch Extraktion, Waschen oder Kristallisation, aufgereinigt werden.

Durch das erfindungsgemäße Reinigungsverfahren wird OPA in sehr hoher, halogenfreier Qualität (>99,5 GC FI%) und in sehr hoher Ausbeute von bis zu über 92% erhalten. Weiters weist erfindungsgemäß gereinigtes OPA eine gleichbleibende Farbe auf, wobei keine Entfärbung mittels Tonsil oder Kohle notwendig ist. Durch das erfindungsgemäße Verfahren werden außerdem Ausbeuteverluste an OPA vermieden.

Weiters zeichnet sich das erfindungsgemäße Verfahren noch durch eine gegenüber dem Stand der Technik verkürzte Reaktionszeit, was auch eine kontinuierliche Fahrweise ermöglicht, aus.

### Beispiel 1:

### Schritt a)

61g (0,25 mol) Tetrachloro-o-xylol (TCX) (Gehalt 99%ig) wurden in einen Autoklaven chargiert und mit 300g (5 mol) Essigsäure versetzt. Dann wurde eine Lösung aus 44g (1,1mol) NaOH und 198g VE-Wasser langsam zugesetzt.
Das Reaktionsgemisch wurde auf 160°C erhitzt und 1 h auf dieser Temperatur gehalten. Reaktionsdruck lag bei 3,8 - 3,9 bar.
Anschließend wurde auf 40°C abgekühlt und er Autoklav entleert (606g =525ml Reaktionslösung).
2 ml Probe wurden gezogen und analysiert:
Ergebnis: 99,43 GC Fl% Roh-OPA

### Extraktion von Roh-OPA:

606g Roh-OPA-Lösung wurden zuerst mit 50 ml VE-Wasser versetzt und dann 4 mal mit 93g MTBE extrahiert.
Die organischen Phasen wurden vereinigt und anschließend am Rotavapor bei 400 - 415 mbar und 34-72°C so lange eingeengt, bis kein Destillat mehr überging.
Es wurden 54g Eindampfrückstand erhalten.

### Schritt b)

Der gesamte Eindampfrückstand wurde mit 375 ml Methanol vorgelegt und 2ml konz. H₂SO₄ dazugegeben, sodass sich ein pH-Wert von 0,5 eingestellt hat.
Nach 2 Stunden bei 50°C wurden 10ml 50%ige NaOH dazugegeben. Das Reaktionsgemisch wurde sodann am Rotavapor eingeengt und anschließend mit 175ml VE-Wasser versetzt und 3x mit 80ml MTBE bei 25°C extrahiert.
Die organischen Phasen wurden vereinigt und am Rotavapor eingeengt.
Auswaage 42,4g
Die Destillation des Roh-Acetals erfolgte bei einer Badtemperatur von 135-140°C, Sumpftemperatur von 113-114°C und einer Kopftemperatur von 110-111°C. Der Druck betrug 8 bis 10mbar.
Auswaage: 41,2g OPA-Dimethoxyacetal (91% der Theorie)

### Schritt c) OPA Dimethoxyacetalspaltung:

Zu 500g VE-Wasser, welches mit Schwefelsäure auf pH 2,0 gestellt wurde, wurden 200g OPA-Dimethoxyacetal zugegeben und der Druck auf ca.150 mbar gesenkt. Anschließend wurde mit dem Aufheizen begonnen. Bei einer Sumpftemperatur von 53°C und 155 mbar wurde mit der Destillatabnahme begonnen. In Summe wurden 270ml Destillat erhalten. IPC-GC Analyse der organischen Phase nach 4 h ergab 99,89 FI.% OPA.
Nach Zugabe von 500ml DIPE wurde der OPA bei 50°C extrahiert. Nach Phasentrennung wurde die organische Phase 2x mit je 100ml VE-Wasser gewaschen.
Die gewaschene hell gelb gefärbte organische Lösung wurde zum Rückfluss erhitzt und bei Normaldruck das Restwasser der org. Phase ausgekreist. In Summe wurden 9,5ml Wasser erhalten.
Anschließend wurde mit dem langsamen Abkühlen begonnen. Bei 42°C setzt die Kristallisation von OPA ein. Es wurde weiter bis auf 15°C abgekühlt!
Der kristallisierte OPA wurde über eine G-2 Fritte abfiltriert, mit 150ml DIPE gewaschen und bei 40-45°C über Nacht im Vakuum getrocknet. Es wurde 91,5g trockener OPA mit einem Gehalt von > 99,8% und 450ml Mutterlauge isoliert. Die Ausbeute betrug 61,5 % der Theorie.
Der Rest an OPA auf 100 % d. Th. befand sich in der Mutterlauge, dem Spaltwasser und dem Waschwasser, die beim nächsten Deacetalisierungsbatch aufgrund der hohen Reinheit und hellen Farbe der Lösungen wieder eingesetzt werden konnten.
Somit war eine quantitative Deacetalisierung möglich.

## Patentansprüche

1. Verfahren zur Herstellung von hochreinem, halogenfreien o-Phthalaldehyd, **dadurch gekennzeichnet, dass**
a) Tetrahalo-o-xylol bei einer Temperatur von 155 - 160°C und einem Druck von 2 bis 5 bar, gegebenenfalls in Anwesenheit eines Phasentransferkatalysators, zu o-Phthalaldehyd hydrolysiert wird, der
b) in einer sauren, alkoholischen Lösung bei einer Temperatur von 0 bis Rückflusstemperatur in das korrespondierende Dialkoxyphthalan überführt und anschließend
c) eine Acetalspaltung durch saure Hydrolyse bei einem pH >1,5 bis pH 7 erfolgt, wodurch hochreiner, halogenfreier o-Phthalaldehyd erhalten wird.

2. Verfahren nach Anspruch 1, **dadurch gekennzeichnet, dass** Schritt a) in einer C₁-C₄-Carbonsäure in Gegenwart einer Base und von Wasser durchgeführt wird.

3. Verfahren nach Anspruch 2, **dadurch gekennzeichnet, dass** die Menge an eingesetzter Carbonsäure bei über 4 bis 20 Moläquivalenten, bezogen auf Tetrahalo-o-xylol, liegt.

4. Verfahren nach Anspruch 1, **dadurch gekennzeichnet, dass** Schritt b) bei einem pH-Wert zwischen 0 und 3 erfolgt.

5. Verfahren nach Anspruch 1, **dadurch gekennzeichnet, dass** Schritt c) bei einem pH-Wert zwischen 1,6 und 2,5 erfolgt.

## Claims

1. Process for preparing high-purity, halogen-free o-phthalaldehyde, **characterized in that**
a) tetrahalo-o-xylene is hydrolyzed at a temperature of 155-160°C and a pressure of from 2 to 5 bar, where appropriate in the presence of a phase-transfer catalyst, to o-phthalaldehyde, which
b) is converted in an acidic alcoholic solution at a temperature of from 0 to the reflux temperature into the corresponding dialkoxyphthalane and, subsequently,
c) an acetal cleavage is effected by acid hydrolysis at a pH >1.5 to pH 7, resulting in high-purity, halogen-free o-phthalaldehyde.

2. Process according to claim 1, **characterized in that** step a) is carried out in a C₁-C₄ carboxylic acid in the presence of a base and of water.

3. Process according to claim 2, **characterized in that** the amount of carboxylic acid employed is above 4 to 20 mole equivalents based on tetrahalo-o-xylene.

4. Process according to claim 1, **characterized in that** step b) takes place at a pH of between 0 and 3.

5. Process according to claim 1, **characterized in that** step c) takes place at a pH of between 1.6 and 2.5.

## Revendications

1. Procédé pour la préparation d'o-phtalaldéhyde de haute pureté, exempt d'halogène, **caractérisé en ce que**
a) du tétrahalogéno-o-xylène est hydrolysé en o-phtalaldéhyde à une température de 155-160°C et une pression de 2 à 5 bars, le cas échéant en présence d'un catalyseur de transfert de phase,
b) on transforme l'o-phtalaldéhyde dans une solution acide, alcoolique à une température de 0 à la température de reflux en dialcoxyphtalane correspondant, puis
c) on réalise une dissociation d'acétal par hydrolyse acide à un pH > 1,5 à pH 7, suite à quoi on obtient l'o-phtalaldéhyde de haute pureté, exempt d'halogène.

2. Procédé selon la revendication 1, **caractérisé en ce que** l'étape a) est réalisée dans un acide C₁-C₄-carboxylique en présence d'une base et d'eau.

3. Procédé selon la revendication 2, **caractérisé en ce que** la quantité d'acide carboxylique utilisé est de plus de 4 à 20 équivalents en mole par rapport au tétrahalogéno-o-xylène.

4. Procédé selon la revendication 1, **caractérisé en ce que** l'étape b) est réalisée à un pH entre 0 et 3.

5. Procédé selon la revendication 1, **caractérisé en ce que** l'étape c) est réalisée à un pH entre 1,6 et 2,5.
